# EUROPÄISCHE PATENTSCHRIFT

(11) **EP 1 124 507 B1**
(45) Veröffentlichungstag und Bekanntmachung des Hinweises auf die Patenterteilung: **15.02.2006**
(21) Anmeldenummer: 00943611.4
(22) Anmeldetag: 12.05.2000
(51) Int. Cl.: A61F 2/36

(54) **GELENKPROTHESEN-SYSTEM**
JOINT PROSTHESIS SYSTEM
SYSTEME DE PROTHESE ARTICULAIRE

(30) Priorität: 14.05.1999 DE 29908391 U
(43) Veröffentlichungstag der Anmeldung: 22.08.2001
(73) Patentinhaber: Merete Management GmbH, 10719 Berlin (DE)
(72) Erfinder: ANAPLIOTIS, Emanuel, D-14195 Berlin (DE)
(74) Vertreter: Christiansen, Henning
(86) Internationale Anmeldenummer: PCT/DE2000/001545
(87) Internationale Veröffentlichungsnummer: WO 2000/069372

(56) Entgegenhaltungen:
- EP-A- 0 498 518
- FR-A- 2 606 628

## Beschreibung

Die Erfindung betrifft ein Gelenkprothesen-System mit einem einen konusförmigen Hals aufweisenden Prothesenschaft, einer Gelenkkugel, welche eine konusförmige Aufnahme aufweist, mit einem länglichen Adapter zwischen Prothesenschaft und Gelenkkugel, wobei die Außenkontur des Adapters an einem Ende der Innenkontur der Gelenkkugel-Aufnahme und die Innenkontur des Adapters an dem gegenüberliegenden anderen Ende der Außenkontur des konusförmigen Halses entspricht.

Ein derartiges Gelenkprothesen-System ist beispielsweise in der älteren DE 198 33 791.4 beschrieben, bei dem Gelenkkugel und konusförmiger Hals des Prothesenschaftes eine Konus-Steckverbindung eingehen. Zur Anpassung der Länge des Prothesenschaftes an die Beinlänge des jeweiligen Patienten ist ein länglicher Adapter vorgesehen, der einerseits mit dem konusförmigen Hals, andererseits mit der Gelenkkugel jeweils eine Konus-Steckverbindung eingeht und auf diese Weise den Hals und damit den gesamten Prothesenschaft in gewünschter Weise verlängert. Da der Adapter auf den Konusabschnitt des Halses aufgesteckt wird, resultiert hieraus eine unerwünschte radiale Verdickung des Halses im Bereich unterhalb der Gelenkkugel, weil der Adapter den Hals dort allseitig umgreift. Da nach der Implantation der Prothese die Gelenkkugel in einem entsprechenden Gegenlager, nämlich einer künstlichen Gelenkspfanne zu liegen kommt, wird durch den radialen Auftrag, der durch den Adapter unterhalb der Gelenkkugel hervorgerufen wird, die Beweglichkeit der Prothese in der Gelenkspfanne eingeschränkt; die Prothese kommt - wegen der adapterbedingten Verdickung des Halses - schon bei kleinerem Winkelausschlag in Berührung mit dem umlaufenden Rand der Gelenkspfanne.

Aus der FR 2 606 628 ist eine Gelenkprothese gemäß dem Oberbegriff von Anspruch 1 mit einem Adapter bekannt, der eine - in Richtung zum Schaft hin - den Außendurchmesser des Konusverlaufs - ausschließlich verringernde - Verjüngung der Außenkontur zur Form- und Gestaltungsanpassung der Konusbasis an den anschließenden Schaft aufweist. Auch ist - wie insbesondere aus der Darstellung der Erfindung in der Figur hervorgeht - die Beweglichkeit der Pfanne weiterhin eingeschränkt, da die Verjüngung innerhalb der Fortsetzung der Kontur der Gelenkkugel - und damit außerhalb des Bewegungsbereichs der Pfanne zu liegen kommt. Des weiteren ist durch die Querschnittsverringerung zum Adapterende hin die Bruchgefahr im Bereich der hier größten Spannungsbeanspruchung des Adapters stark heraufgesetzt.

Aufgabe der Erfindung ist es, ein Gelenkprothesen-System der eingangs genannten Art derart weiterzubilden, daß die Beweglichkeit der Prothese in einer künstlichen Gelenkspfanne unter Gewährleistung der Festigkeit des Adapters im Endbereich bei dessen Einsatz nur wenig beeinträchtigt ist.

Diese Aufgabe wird bei dem Gelenkprothesen-System der eingangs genannten Art erfindungsgemäß dadurch gelöst, daß der Adapter unterhalb der Gelenkkugel eine um seine Längsachse umlaufende Umfangsnut an der Außenkontur aufweist, wobei die Außenkontur der Gelenkkugel in ihrer Fortsetzung in der ringförmigen Umfangsnut des Adapters mündet.

Die Vorteile der Erfindung liegen insbesondere darin, dass auf der Außenfläche des Adapters, unmittelbar unterhalb der Gelenkkugel oder in geringem Abstand von der Gelenkkugel eine Umfangsnut umläuft, welche die adapterbedingte Verdickung des Halses dort reduziert, wo die - implantierte - Prothese gegen den Rand der künstlichen Gelenkspfanne anschlägt. Da die Umfangsnut die adapterbedingte Verdickung des Halses entsprechend reduziert, ist die Auslenkung der Prothese bis der Prothesenhals an den Rand der Gelenkspfanne anschlägt, entsprechend vergrößert. Die Beweglichkeit der mit dem Gelenkkopf versehenen Prothese in der Gelenkspfanne ist entsprechend vergrößert.

Gemäß einer bevorzugten Ausführungsform der Erfindung besitzt die Umfangsnut - in Richtung der Längsachse des Adapters gemessen - eine vorgegebene axiale Breite und quer zur Längsachse eine vorgegebene radiale Tiefe. Die Seitenwände der Umfangsnut gehen mit vorgegebenen Rundungsradien in die Basis der Umfangsnut über, um sicherzugehen, dass an diesen Stellen keine Spitzenlasten auftreten, wenn die Konus-Steckverbindungen zwischen Hals und Adapter bzw. Adapter und Gelenkkugel mit Kräften beaufschlagt sind.

Gemäß einer bevorzugten Ausführungsform der Erfindung ist der Adapter hülsenförmig ausgebildet und besitzt an seinem der Gelenkkugel zugewandten Ende einen Boden, der mit einer Profilöffnung versehen ist und das Ansetzen eines Abziehwerkzeuges erlaubt.

Erfindungsgemäß werden jeweils Adapter verschiedener axialer Länge verfügbar gehalten, um nach den operativen Gegebenheiten den Adapter mit geeigneter Länge auswählen zu können, der den Hals des Prothesenschaftes entsprechend den Anforderungen des Patienten verlängert. Vorteilhafte Weiterbildungen der Erfindung sind durch die Merkmale der Unteransprüche gekennzeichnet.

Im folgenden werden bevorzugte Ausführungsformen der Erfindung anhand der Zeichnungen näher erläutert. Es zeigen:
- Fig. 1: einen Schnitt durch eine erste Ausführungsform des Gelenkprothesen-Systems, wobei das distale Ende des Prothesenschaftes weggebrochen ist;
- Fig. 2: eine der Fig. 1 entsprechende Darstellung mit einem längeren Adapter und
- Fig.3: eine den Fig. 1 und 2 entsprechende Schnittdarstellung einer weiteren Ausführungsform des Gelenkprothesen-Systems.

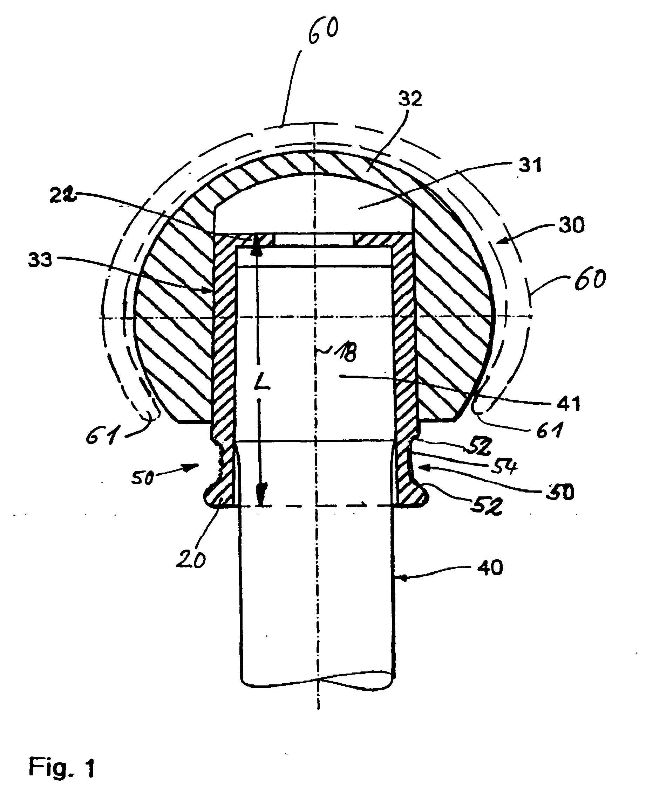

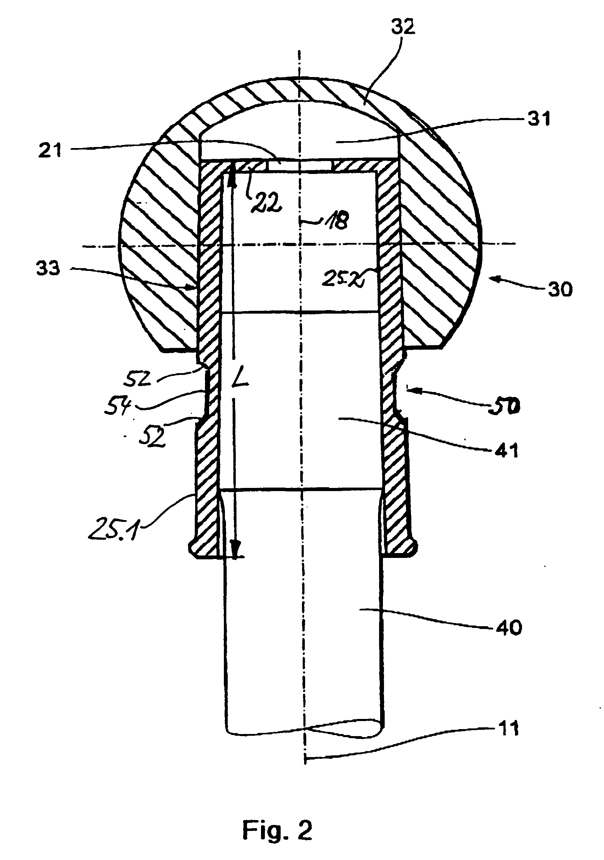

Die Fig. 1 und 2 zeigen einen Schnitt durch eine Ausführungsform des Gelenkprothesen-Systems, bei dem das distale Ende des Prothesenschaftes 40 weggebrochen ist. An dem Prothesenschaft 40 schließt sich ein konusförmiger Hals 41 an, auf dem ein länglicher Adapter 20 aufgesteckt ist, dessen Längsachse 18 mit der Längsachse 11 des konusförmigen Halses 41 fluchtet. Der Adapter ist in der in den Fig. 1 und 2 dargestellten Ausführungsform als Hülse ausgebildet und besitzt eine Außenkontur 25.1, die am oberen Ende des Adapters 20 mit der Innenkontur der Gelenkkugel-Aufnahme 33 eine Konus-Steckverbindung bildet. Die Innenkontur 25.2 des Adapters 20 bildet an dem gegenüberliegenden, unteren Ende des Adapters eine konusförmige Bohrung, die mit der Außenkontur des konusförmigen Halses 41 ebenfalls eine Konus-Steckverbindung eingeht. Wie aus einer Gegenüberstellung der Fig. 1 und 2 ersichtlich ist, werden für einen Prothesenschaft mehrere Adapter 20 mit unterschiedlicher Länge L verfügbar gehalten, die - je nach den Gegebenheiten des Patienten - auf den konusförmigen Hals 41 aufsetzbar sind und der unterschiedlichen Verlängerung des Halses 41 dienen.
Über dem Adapter 20, bzw zwischen Boden 22 und Gelenkkugelboden 31 entsteht ein Leerraum 31, der je nach Passung der Konuskonturen der Gelenkkugel 30 zu der Außenkotur des Adapters 20, größer oder kleiner sein kann.

In geringem Abstand unter der Gelenkkugel 30 ist eine um die Längsachse 18 des Adapters 20 umlaufende Umfangsnut 50 vorgesehen, die ringförmig am Außenumfang herumläuft und eine Basis 54 sowie zwei Seitenwände 52 besitzt. Die Umfangsnut besitzt eine vorgegebene axiale Breite, gemessen in Längsrichtung der Achse und eine vorgegebene radiale Tiefe, gemessen quer zur Längsachse 18. Die Umfangsnut 50 liegt an einer Stelle, an der die künstliche Gelenkpfanne 60, hier gestrichelt dargestellt, bei entsprechender Auslenkung der Prothese 40 den Adapter 20 berührt. Durch die Umfangsnut 50 ist sichergestellt, dass der Auslenkungswinkel, um den die Prothese in der Pfanne 60 ausgelenkt werden kann, vergrößert ist, bedingt durch das Eintauchen der Enden 61 der Gelenkpfanne 60 in die Umfangsnut 50, wodurch sich eine erhöhte Beweglichkeit zwischen Gelenkpfanne 60 und Prothese 40 ergibt.

Der in den Fig. 1 und 2 dargestellte Adapter 20 besitzt an seinem der Gelenkkugel 30 zugewandten Ende einen Boden 22, in den eine Profilöffnung 21 eingearbeitet ist, um ein Abziehwerkzeug ansetzen zu können.

Wie den Fig. 1 und 2 entnehmbar ist, gehen die Seitenwände 52 der Umfangsnut 50 mit vorgegebenen Rundungsradien in die Basis 54 der Umfangsnut über, um Spannungsspitzen zu vermeiden, welche bei einem winkeligen Übergang der Seitenwände 52 in die Basis 54 entstehen könnten.

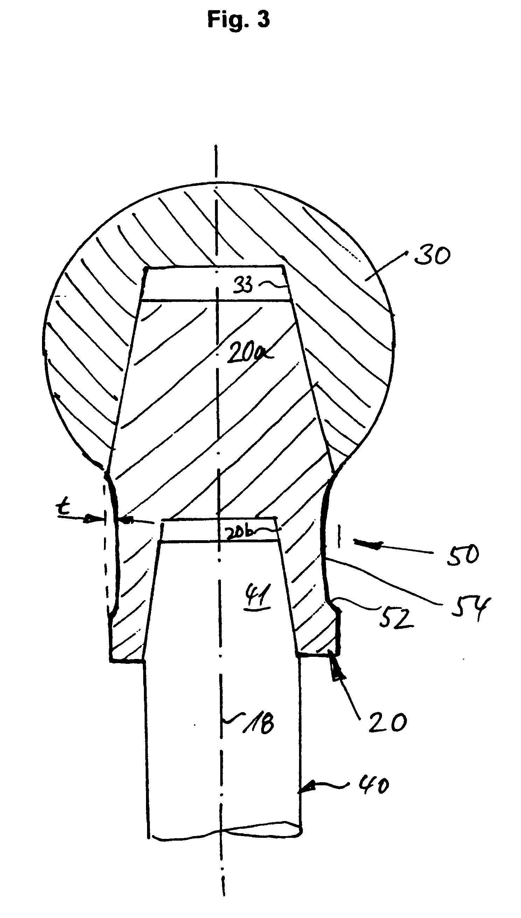

Fig. 3 zeigt eine weitere Ausführungsform der Erfindung, bei welcher der Adapter 20 aus Vollmaterial besteht und wiederum mit der Aufnahme 33 der Gelenkkugel 30 sowie mit dem konusförmigen Hals 41 eine Konus-Steckverbindung eingeht, wobei jedoch in der dargestellten Ausführungsform der am oberen-Ende in die Gelenkkugel 30 hineinragende Konus 20a aus vollem Material besteht und am unteren Ende des Adapters 20 eine Konusbohrung 20b eingearbeitet ist, die mit dem konusförmigen Hals 41 zusammenpasst.

Am Außenumfang des Adapters 20 ist eine um die Längsachse 18 umlaufende Umfangsnut 50 vorgesehen, deren radiale Tiefe t so bemessen ist, dass die mit der Gelenkkugel versehene Prothese ein ausreichendes Bewegungsspiel in der Gelenkpfanne hat.

### Liste der Bezugszeichen:

- 11: Längsachse
- 20: Adapter
- 20a: Konus
- 20b: Konusbohrung
- 21: Profilöffnung
- 22: Boden
- 25.1: Außenkontur
- 25.2: Innenkontur
- 30: Gelenkkugel
- 31: Leerraum
- 32: Gelenkkugelboden
- 33: Gelenkkugelaufnahme
- 40: Prothesenschaft
- 41: Hals
- 50: Umfangsnut
- 52: Seitenwände
- 54: Basis
- 60: Gelenkpfanne

## Patentansprüche

1. Gelenkprothesen-System (50) mit einem einen konusförmigen Hals (41) aufweisenden Prothesenschaft (40), einer Gelenkkugel (30), welche eine konusförmige Aufnahme (33) aufweist, mit einem länglichen Adapter (20) zwischen Prothesenschaft und Gelenkkugel, wobei die Außenkontur (25.1) des Adapters (20) an einem Ende der Innenkontur der Gelenkkugel-Aufnahme (33), und die Innenkontur (25.2) des Adapters (20) an dem gegenüberliegenden anderen Ende der Außenkontur des konusförmigen Halses (41) entspricht, **dadurch gekennzeichnet, daß** der Adapter (20) unterhalb der Gelenkkugel (30) eine um seine Längsachse (18) umlaufende, vom unteren Adapterende beabstandete Umfangsnut (50) an der Außenkontur (25.1) aufweist und die Außenkontur der Gelenkkugel (30) in ihrer Fortsetzung in der ringförmigen Umfangsnut (50) des Adapters (20) mündet.

2. Gelenkprothesen-System nach Anspruch 1, **dadurch gekennzeichnet, daß** die Umfangsnut (50) eine vorgegebene axiale Breite, gemessen in Richtung der Längsachse (18) des Adapters (20) und eine vorgegebene radiale Tiefe, quer zur Längsachse (18) gemessen, besitzt.

3. Gelenkprothesen-System nach einem der vorstehenden Ansprüche, **dadurch gekennzeichnet, daß** zur Verwirklichung unterschiedlich langer Halsabschnitte (41) Adapter unterschiedlich axialer Länge L verfügbar sind.

4. Gelenkprothesen-System nach einem der vorstehenden Ansprüche, **dadurch gekennzeichnet, daß** der Adapter (20) hülsenförmig ausgebildet ist und an seinem der Gelenkkugel (30) zugewandten Ende einen Boden (22) aufweist.

5. Gelenkprothesen-System nach Anspruch 4, **dadurch gekennzeichnet, daß** der Boden (22) mit einer Profilöffnung (21) zum Ansetzen eines Abziehwerkzeuges versehen ist.

6. Gelenkprothesen-System nach einem der vorstehenden Ansprüche, **dadurch gekennzeichnet, daß** die Seitenwände (52) der Umfangsnut (50) mit vorgegebenen Rundungsradien in die Basis (54) der Umfangsnut (50) übergehen.

## Claims

1. Joint prosthesis system (50) having a prosthesis shank (40) with a conical neck (41), a joint ball (30) with a conical admission (33) and an oblong adapter (20) interposed between the prosthesis shank and the joint ball, where the outer contour (25.1) of the adapter (20) at one end corresponds to the internal contour of the joint ball admission (33), and the internal contour (25.2) of the adapter (20) at the opposite end corresponds to the outer contour of the conical neck (41), ***characterized by** the fact* that the adapter (20) exhibits a circumventing groove (50) at the outer contour (25.1), circumventing its longitudinal axis (18), beneath the joint ball (30) distant from the lower end of the adapter.

2. Joint prosthesis system according to claim 1, ***characterized by** the fact* that the circumventing groove (50) has a given axial width, measured in the direction of the longitudinal axis (18) of the adapter (20) and a given radial depth, measured in a transversal direction with respect to the longitudinal axis (18).

3. Joint prosthesis system according to one of the preceding claims, ***characterized by** the fact* that for the implementation of neck sections of different lengths (41) of adapters different axial lengths L are provided.

4. Joint prosthesis system according to one of the preceding claims, ***characterized, by** the fact* that the adapter (20) is capsule-shaped having a closed bottom (22) at its end next to the joint ball (30).

5. Joint prosthesis system according to claim 4, ***characterized, by** the fact* that the bottom (22) has a profiled aperture (21) to receive a retraction tool.

6. Joint prosthesis system according to one of the preceding claims, ***characterized, by** the fact* that the side walls (52) of the circumventing groove (50) smooth into the base (54) of the circumventing groove (50).

## Revendications

1. Système de prothèse de joint (50) marqué avec une tige de prothèse (40), d'une boule de joint (30) qui montre une admission (33) conique, montrant un col (41) conique, avec un adaptateur (20) oblongue située entre la tige de prothèse et la boule de joint, auquel cas les grandes lignes extérieures (25.1) de l'adaptateur (20) à une fin des grandes lignes à l'intérieur de l'admission de boule de joint (33), et grandes lignes à l'intérieur (25.2) de l'adaptateur (20) à l'opposé correspondent à l'autre fin des grandes lignes extérieures du col (41) conique, **caractérisée en ce que** l'adaptateur (20) montre une cannelure d'ampleur (50) que orbite autour de son axe longitudinal (18) aux grandes lignes extérieures (25.1) au-dessous de la boule de joint (30) dans une distance de la fin d'adaptateur inférieure.

2. Système de prothèse selon de revendication 1, **caractérisée en ce que** la cannelure d'ampleur (50) possède une largeur axiale alléguée, mesuré en direction de l'axe longitudinal (18) de l'adaptateur (20) et une profondeur radiale alléguée, à l'axe longitudinal (18) mesuré de travers.

3. Système de prothèse de joint selon une de revendications précédentes, **caractérisée en ce que** pour réaliser, les sections de col (41) différemment longues des adaptateurs de longueur L différemment axiale sont disponibles.

4. Système de prothèse de joint selon une de revendications précédentes, **caractérisée en ce que** l'adaptateur (20) est formé capsulaire et montre à sa boule de joint (30) un sol (22).

5. Système de prothèse de joint selon revendication 4, **caractérisée en ce que** le sol (22) est fourni avec une ouverture de profil (21) pour la fixation d'un outil enlever.

6. Système de prothèse de joint selon une de revendications précédentes, **caractérisée en ce que** les parois latérales (52) de la cannelure d'ampleur (50) avec des rayons d'arrondissement allégués passent dans la base (54) de la cannelure d'ampleur (50).
